# EUROPEAN PATENT SPECIFICATION

(11) **EP 4 311 582 B1**
(45) Date of publication and mention of the grant of the patent: **01.10.2025**
(21) Application number: 22382721.3
(22) Date of filing: 27.07.2022
(51) Int. Cl.: A63B 47/00, G01L 17/00, G01N 3/14, G01B 5/00, G01B 5/30, G01M 99/00, G01N 33/00

(54) **TESTING THE STATUS OF TENNIS BALLS**
PRÜFUNG DES ZUSTANDES VON TENNISBÄLLEN
TEST DE L'ÉTAT DE BALLES DE TENNIS

(43) Date of publication of application: 31.01.2024
(73) Proprietor: Jarmen Inversiones Activas S.L.U, 08021 Barcelona (ES)
(72) Inventor: ARMENGOL ALMELA, Javier, 08022 BARCELONA (ES)
(74) Representative: ZBM Patents - Zea, Barlocci & Markvardsen

(56) References cited:
- US-A- 4 114 350
- US-A- 5 760 312
- US-A1- 2007 275 798
- US-A1- 2020 254 313

## Description

The present disclosure relates to methods and devices for testing the status of a tennis ball.

### BACKGROUND

Tennis balls must comply with certain specifications defined by the International Tennis Federation in order to be approved for playing under the rules of tennis. These specifications relate to mass, size, rebound, deformation and color of tennis balls. For example, the mass approved is between 56.0 g and 59.4 g and the size of the approved tennis ball may vary between 6.54 cm and 7.30 cm. According to these specifications, rebound when tennis ball falls down from 254 cm may be between certain values, e.g. between 122 cm and 151 cm for some types of tennis balls. Variations in the internal pressure of the tennis ball may affect the rebound. Deformation is also regulated by these specifications and must be between 0.56 cm and 1.08 cm.

Furthermore, the International Padel Federation has ruled similar constraints for tennis balls approved to be used for official competitions of padel. The diameter of the tennis ball approved for padel should measure between 6.35 and 6.77 cm and should weigh between 56.0 and 59.4 g. When the approved tennis ball for padel falls down onto a hard surface from a height of 254 cm, the bounce is expected to be between 135 and 145 cm.

The deformation of a tennis ball to be measured may be very small. In reason of that, detecting by eye or by pressing with the fingers or hand the deformation of the ball is not trivial. Consequently, a precise measurement of the deformation is difficult to achieve.

Furthermore, the internal pressure of tennis balls may vary over time. Tennis balls generally lose their original characteristics after just one hour of use. This degradation goes on and, after several matches, tennis balls are commonly discarded as do not meet the expected requirements. Tennis balls may thus be deformed after short use. Accordingly, the response of the ball during a tennis match may substantially vary after a few matches. Players may wish to know in advance how many matches the tennis ball has been used in order to know the expected response of the tennis ball in upcoming tennis or paddle matches.

Devices to measure the shape of the tennis balls may be used to test if the tennis ball is in condition for a tennis game or how many times the tennis ball has been used

Document US2020254313A1 discloses a device for testing a status of a tennis ball comprising all the technical features set out in the preamble of claim 1.

However, these devices are generally heavy and difficult to use. In these devices, a force may be applied to the tennis ball and the deformation caused by this force is measured. Depending on the deformation experienced by the tennis ball, the status of the tennis ball may be determined. However, the force applied to the tennis ball depends on the user. Accordingly, the deformation experienced by the tennis ball may vary from user to user.

The present disclosure provides examples of systems and methods that at least partially resolve some of the aforementioned disadvantages.

### SUMMARY OF THE INVENTION

The present invention is directed to a device for testing a status of a tennis ball according to claim 1 and to a method for testing a status of a tennis ball according to claim 15. Additional features and embodiments of the invention are defined in the dependent claims.

### SUMMARY OF THE DISCLOSURE

In a first aspect, a device for testing the status of a tennis ball is disclosed. The status of the tennis ball refers to the condition of the ball and the capability thereof to be used for a tennis game, a tennis training, a padel game, or the like. In this disclosure, the internal pressure of the tennis ball reflects the status of the tennis ball. In this disclosure, a tennis ball refers to tennis balls used in tennis and to tennis balls used in padel.

The device comprises a base and a top element movably connected to the base. The device comprises a cavity between the base and the top element configured to receive the tennis ball. The device further comprises an elastic traction connector movably connecting the base to the top element, such that the top element is movable between an insertion position for inserting the tennis ball into the cavity and a pressure position in which the top element forces the ball against the base by the effect of a traction exerted by the elastic traction connector. In addition, the device comprises a measurement instrument for measuring a distance between the base and the top element.

The base and the top element define the shape of the cavity. The dimension and/or the shape of the cavity may be configured to receive a tennis ball. A tennis ball can thus be inserted into the cavity formed between the base and the top element.

The length of the elastic traction connector is lower at rest than when working at a stretched position: at the insertion position and at the pressure position. The elastic traction connector tends to move the top element towards the base. Therefore, when the elastic traction connector is pulled, a tensile reaction force is exerted in a direction of an axis of the elastic traction connector. The elastic traction connector causes the top element to move between an insertion position for inserting the ball into the cavity and a pressure position. In the pressure position, the elastic traction connector applies a force to the top element towards the base. The top element forces the ball against the base by the effect of the traction exerted by the elastic traction connector. The insertion position may be the position in which the distance between the top element and the base is maximum or at least larger than the diameter of the ball. The ball may thus be inserted into the cavity. A user may pull the top element to move the top element away from the base so as to reach the insertion position. The size of the cavity can thus vary from an insertion position to a pressure position. The elastic traction connector is thus stretched when the top element is in the insertion position.

While the top element is in the insertion position, the distance between the base and the top element is independent of the presence of the ball. When the ball is inserted into the cavity, the top element moves toward the base to reach the pressure position. At the pressure position, the fact that the top element forces the ball against the base may cause a deformation of the ball.

Therefore, the measurement instrument may indicate the deformation of the ball. The result of the measurement aims at precisely knowing the status of the tennis ball. The expected response of the tennis ball in future tennis or paddle games may thus be quickly and easily determined. Furthermore, determining the status of the tennis ball may be used for differentiating between a ball in good conditions to be used in a tennis or in a padel game and a ball so stressed to not be usable for tennis games.

In this context, if the top element forcing the ball against the base results in a deformation of the ball inferior to a certain amount, the ball is in a good state Therefore, the ball may be used in a game. On the contrary, if the measurement instrument indicates a deformation of the ball higher than a certain maximum deformation, the ball is not in a condition to be used for a game anymore.

The deformation of the tennis ball is an indicator of its status of the tennis ball. Measuring the distance between the top element and the base is a direct expression of the status of the tennis ball. In fact, for tennis balls of the same model, the distance between the base and the top element is smaller for balls more deformed than for balls less deformed. Therefore, measuring the distance between the top element and the base is a quantification of the internal pressure of the tennis ball.

According to this aspect, the force applied to the tennis ball depends on the elastic traction connector. No external forces are required to press the tennis ball. Accordingly, the force applied to the tennis ball is independent of the user. A more uniform force may thus be applied. Consequently, the precision of the deformation measurement may be enhanced. A more robust, simpler, and cheaper device may be obtained while the measurement of the deformation of tennis balls is more reliable. Transportation of the device is further improved.

In a second aspect, a method for testing the status of a tennis ball is disclosed. The method comprises providing a device according to any of the examples of the present disclosure. The method comprises moving the top element to the insertion position by stretching the elastic traction connector in order to create a cavity big enough to insert the tennis thereto. Further, the method comprises inserting the tennis ball into the cavity. The method comprises releasing the top element to move the top element from the insertion position to the pressure position by the effect of the traction exerted by the elastic traction connector, such that moving the top element to the pressure position forces the ball against the base. This forcing the ball against the base may cause the ball to deform. Additionally, the method comprises measuring a distance between the top element and the base.

Advantages derived from this aspect may be similar to those mentioned regarding the previous aspect.

### BRIEF DESCRIPTION OF THE DRAWINGS

Non-limiting examples of the present disclosure will be described in the following, with reference to the appended drawings, in which:
Figure 1A is a schematic representation of a cross-sectional view of a device in an insertion position according to an example of the present disclosure.
Figure 1B is a schematic representation of a cross-sectional view of the device of figure 1A in a pressure position.
Figure 2 is a schematic representation of a cross-sectional view of a base of a device according to an example of the present disclosure.
Figure 3 is a schematic representation of a cross-sectional view of a top element of a device according to an example of the present disclosure.
Figure 4 is a schematic representation of a front view of a device according to an example of the present disclosure.
Figure 5 is a top view representation of a device according to an example of the present disclosure.

### DETAILED DESCRIPTION OF EXAMPLES

In the figures herein disclosed, the same reference signs have been used to designate matching elements.

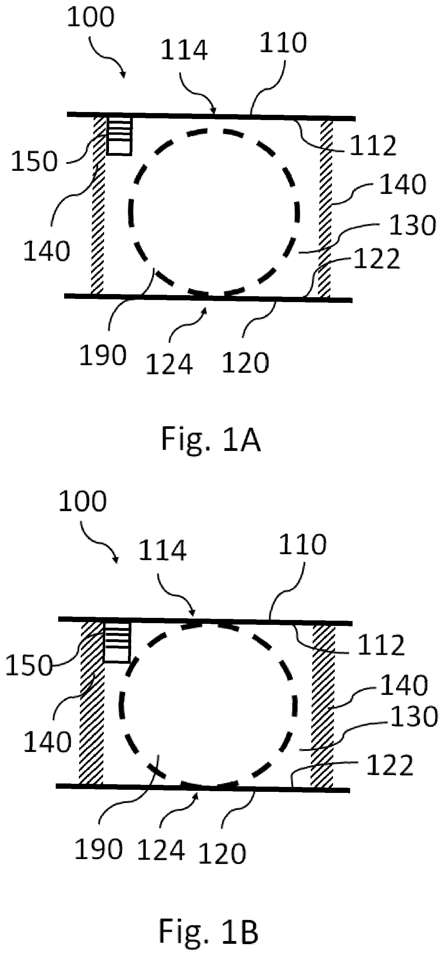

Fig. 1A and 1B schematically represent a cross-sectional view of a device 100 for testing the status of a tennis ball according to an example of the present disclosure. The device 100 comprises a top element 110, a base 120 and a cavity 130 between the top element 110 and the base 120. The cavity 130 is configured to receive the tennis ball 190. The device 100 comprises an elastic traction connector 140 movably connecting the base 120 to the top element 110.

The elastic traction connector 140 allows the top element 110 to move between an insertion position (Figure 1A) for inserting the tennis ball 190 into the cavity 130 and a pressure position (Figure 1B) in which the top element 110 forces the tennis ball 190 against the base 120 by the effect of a traction exerted by the elastic traction connector 140. The fact that the top element 110 is configured to move between the insertion position and the pressure position implies that a dimension of the cavity 130 corresponding to a distance between the top element 110 and the base 120 may change.

The elastic traction connector 140 may be moved from a rest or relax position to the insertion position and then to a pressure position. In the rest position, the elastic traction connector does not generate a traction force. The elastic traction connector is stretched in both the insertion position and the pressure position. However, the degree of stretch is greater in the insertion position than in the pressure position.

The cavity 130 is configured to receive the tennis ball 190 when the top element 110 is in the insertion position. When the top element 110 is in the insertion position the dimension of the cavity 130 may be bigger than when the top element 110 is in the pressure position. In the insertion position, the distance between the top element 110 and the base 120 is greater than a diameter of the tennis ball 190, e.g. greater than 7.30 cm.

In figure 1B, the top element 110 is in the pressure position. The distance between the top element 110 and the base 120 in the insertion position is greater than in the pressure position. In the pressure position, both the top element 110 and the base 120 abut the tennis ball 190. The top element 110 presses the tennis ball 190 against the base 120 to deform the tennis ball 190.

When the elastic traction connector 140 is in the rest position, the distance between the top element 110 and the base 120 is smaller than when at the insertion position and at the pressure position.

The elastic traction connector 140 of these figures comprises two connector springs. According to other examples, the elastic traction connector 140 may comprise three, four, six traction springs or a multiple thereof. In these figures, the elastic traction connector 140 is stretched and tends to return to the rest position.

The springs may be configured to provide a uniform traction force when stretched. For example, the springs may provide a traction force between 75 N and 80 N.

In some examples, the elastic traction connector 140 comprises an elastic band or a plurality of elastic bands. The elastic band may encircle the top element and the base, such that when pulling the top element away from the base, inserting the ball into the cavity is allowed. The elastic band may push the top element against the base when releasing the elastic band from the insertion position. Therefore, no external forces are required to move the top element towards the base but only the reaction force of the elastic traction connector 140.

In some examples, the elastic traction connector 140 may comprise a plurality of connector springs and an elastic band or a plurality of elastic bands.

The device of these figures also comprises a measurement instrument 150 for measuring a distance between the base 120 and the top element 110. In this example, the measurement instrument 150 is a ruler. Therefore, the distance measured may be a direct measurement of the deformation of the tennis ball.

Tennis balls may then be classified depending on the status determined by using the measurement instrument. For example, tennis balls may be classified or ranked into groups depending on the expected use. Depending on the expected use, the requirements of the tennis balls may be different. For example, more deformed tennis balls may be used in training activities because the balls are generally slower. On the other hand, the requirements of tennis balls in professional or semi-professional games are more demanding.

In some examples, ranking or sorting out the tennis balls may take into account the initial or manufacturing diameter, i.e. the diameter before any use. Tennis balls of different manufacturing diameters are thus ranked separately. Tennis balls can thus be separated by manufacturers and then ranked according to the status of the internal pressure.

In some examples, the measurement instrument 150 may comprise magnifying glasses in order to magnify the measurement of the deformation of the tennis ball inserted in the device 100. Therefore, it may be possible to read the measurement more accurately because the measurement will be magnified by the magnifying glasses.

In some examples, the measurement instrument 150 may comprise an indicator to indicate if the distance between the top element 110 and the base 120 is equal to or smaller than a measuring threshold. For example, this measuring threshold may be a minimum acceptable diameter for a tennis ball. The status of the tennis ball may thus be easily determined.

In some examples, the measurement instrument may comprise a plurality of divisions. Tennis balls may be classified depending on the division reached when the top element presses the tennis ball against the ball.

In some examples, the measurement instrument may be a numbered scale. For example, the numbered scale may be from 1 to 10. The numbers of the numbered scale may increase from the top element to the base. The number 10 is thus arranged closer to the base than the number 1 of the numbered scale. For example, when the top element 110 stops at the number 1, the tennis ball is in perfect condition. When the top element 110 is stopped at lower parts of the numbered scale, this is an indication that the tennis ball has loose internal pressure. For example, if the top element 110 is stopped or reaches the number 10 of the numbered scale, this is an indication that the tennis ball is no longer eligible for a tennis or paddle ball. Thus, tennis balls can be grouped with similar technical responses, e.g. balls indicating numbers 1 - 2, balls indicating numbers 3 - 4, etc. In some examples, instead of a numbered scale from 1 to 10, the numbered scale may comprise any other number of divisions.

In some examples, when the device is used for measuring tennis balls having substantially the same manufacturing diameter, the measuring indicator may be e.g. a colored label. The colored label may be visible when the distance is equal to or smaller than the measuring threshold. This may involve that the deformation exceeds the expected deformation. As a result, if the color label is visible, the tennis ball is not apt to be used for a tennis or a padel game. Alternatively, the colored label may be visible when the deformation of the tennis ball is under an expected range. In these examples, the colored label indicates that the tennis ball is fit for use. In other examples, the indicator is a plurality of color labels. The plurality of color labels may be used for classifying the tennis balls according to their status for each manufacturer.

The base 110 of this example comprises a base cavity surface 122 facing the cavity 130. The base cavity surface 122 thus faces the ball 190 when inserted into the cavity 130. The base cavity surface 122 of these figures comprises a base engaging portion 124 to contact or engage a portion of the ball 190 when the tennis ball 190 is in the cavity of the device. In these figures, the base engaging portion 124 is substantially flat. In other examples, the base engaging portion may comprise a recess to accommodate the tennis ball.

In these figures, the top element 110 comprises a top element cavity surface 112 facing the cavity 130. The top element cavity surface 112 of this example comprises a top element engaging portion 114 configured to contact or engage a portion of the ball 190. In figures 1A - 1B, the top element engaging portion 114 is substantially flat. In further examples, the top element engaging portion may comprise a top element protrusion extending towards the cavity.

In some examples, the device comprises an anchor configured to anchor the base to a substantially flat surface. The anchor may comprise a suction pad or cup, a clamp and/or a screw. The presence of the anchor enables keeping the device in a specific position without the need of holding the device. In this way, a user who wants to check the status of a tennis ball can use one hand to pull the top element and the other hand to insert the tennis ball into the cavity. Therefore, the presence of the anchor simplifies the use of the device. The anchor may be arranged at the base, e.g. at a side opposite to the base cavity surface.

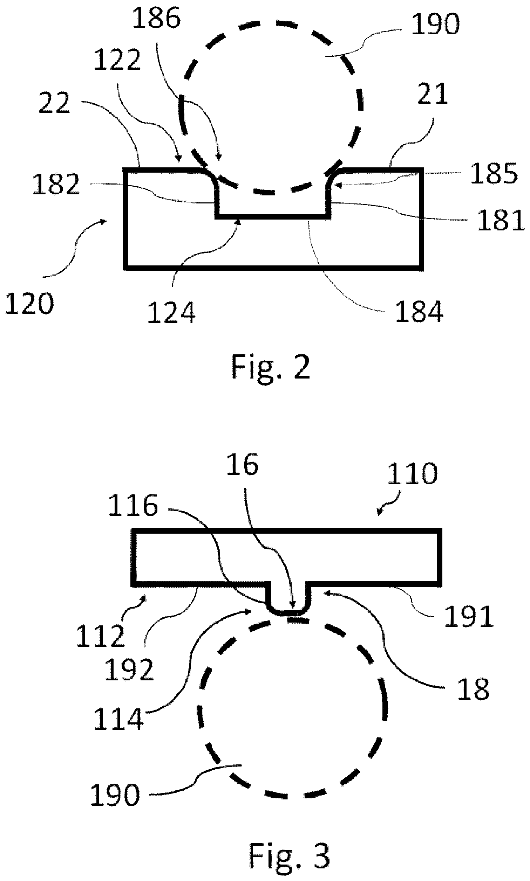

Figure 2 is a schematic representation of a cross-sectional view of a base of a device according to an example of the present disclosure. In this figure, the base 120 comprises a base cavity surface 122 configured to face the cavity defined between the base and the top element (not shown in this figure).

The base cavity surface 122 of this figure comprises a base engaging portion 124 configured to engage a portion of the tennis ball 190 that faces the base cavity surface 122. The base engaging portion 124 of this figure comprises a recess to accommodate the tennis ball 190. The base engaging portion 124 is configured to engage or fit the tennis ball in one or more points thereof such that the tennis ball may be easily retained within the cavity. Therefore, the base engaging portion 124 may allow a precise positioning and retention of the tennis ball into the cavity of the device.

The base cavity surface 122 of this example comprises a first flat wall 21 and a second flat wall 22. The base engaging portion 124 is arranged between the first flat wall 21 and the second flat wall 22. In this example, the base engaging portion 124 comprises a rectangular cross-section. The base engaging portion 124 comprises a first side wall 181 and a second side wall 182. The first side wall 181 extends from a bottom wall 184 of the base engaging portion 124 to the first flat wall 21. In this figure, a first edge 185 connects the first flat wall 21 to the first side wall 181. The second side wall 182 extends from the bottom wall 184 to the second flat wall 22 through a second edge 186. In this example, the tennis ball 190 is contacted by the first edge 185 and the second edge 186. The edges 185 and 186 of this example are rounded to fit the external shape of the tennis ball 190.

In this example, the recess of the base engaging portion 124 extends substantially constant along a longitudinal direction (perpendicular to the paper). In other examples, the shape of the recess may vary along a longitudinal direction of the base. The length of the bottom wall 184 may be smaller at the ends of the recess. The recess may thus be adapted to the shape of the tennis ball. The recess may comprise a circular shape when viewed from the cavity.

In other examples, the recess of the base engaging portion 124 may comprise a concave shape configured to fit a portion of the tennis ball. In these examples, the recess may comprise a curved cross-section that may vary along the longitudinal direction of the base. The recess may thus be a portion of a semi-sphere. This region of the semi-sphere may thus have a shape corresponding to a portion of the tennis ball. The recess may comprise a circular shape when viewed from the cavity.

The device may comprise an adjusting element configured to retain the ball within the base engaging portion. In some examples, the adjusting element may be arranged at the base. The adjusting element may be e.g. deformable protrusion or an adjusting strip configured to surround a portion of the ball. The adjusting strip may be connected to the first side wall and/or to the second side wall. The adjusting element may deform under the pressure exercised by the tennis ball so as to secure the tennis ball in the base engaging portion. Accordingly, the retention of the ball into the base may be improved. In addition, the adjusting element allows using the device to test tennis balls with relatively small diameter variations.

Fig. 3 is a schematic representation of a cross-sectional view of the top element 110 according to an example of the present disclosure. In this example, the top element 110 comprises a top element cavity surface 112 facing the cavity. As a result, the top element cavity surface 112 points at the inside of the device. The top element cavity surface 112 of this figure comprises a top engaging portion 114 configured to engage a portion of the tennis ball 190 facing the top element cavity surface 112.

The top engaging portion 114 of fig. 3 comprises a top element protrusion 116 configured to contact or abut a portion of the tennis ball 190 facing the top element cavity surface 112. The presence of the top element protrusion 116 allows the pressure to be concentrated in a central portion of the ball. Deformation of the tennis ball when the test is performed is thus increased. The top element protrusion 116 may contact a specific portion of the ball so that the tennis ball is forced against the base cavity surface in a repeatable manner. Therefore, the top element protrusion may improve the repeatability of the measurement.

The top element protrusion 116 of this example extends from the top element cavity surface 112 towards the base cavity surface. The top element protrusion 116 comprises a proximal end 18 to the element cavity surface 112 and a distal end 16 pointing at the cavity. In this example, the element cavity surface 112 comprises a first wall 191 and a second wall 192. The first and the second wall of this example are substantially flat; however, in other examples, these walls may be inclined.

The top element protrusion 116 of this figure is arranged between the first side wall 191 and the second side wall 192. In this example, the tennis ball 190 is pressed by the top element protrusion 116. A distal end 16 of the top element protrusion 116 is configured to engage a portion of the tennis ball. For example, the distal end 16 may comprise a concave shape.

In some examples, the distal end 16 of the top element protrusion has a squared cross-section. The sides of the squared cross-section of the distal end 16 may be between 5 mm and 25 mm, e.g. 10 mm and 20 mm.

In other examples, the top element protrusion may have a circular cross-section, e.g. substantially constant from the proximal end 18 to the distal end 16. The diameter of the top element protrusion may be between 5 mm and 25 mm, e.g. 10 mm and 20 mm.

The device may comprise a top element and a base according to any of the examples herein disclosed. For example, the device may comprise a top element according to the example of fig. 3 and a base according to the example of fig. 2.

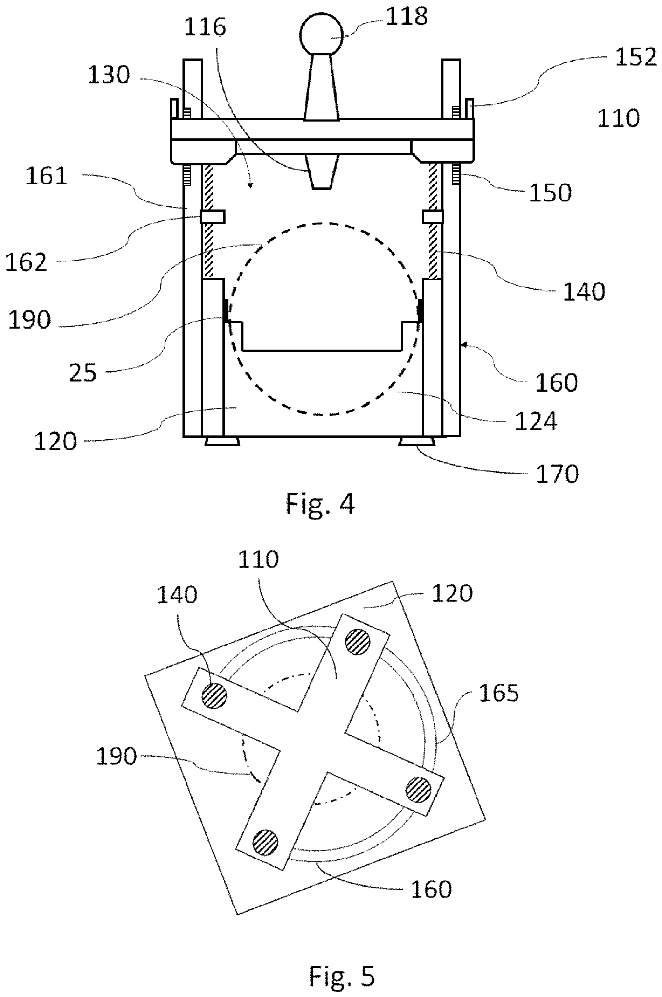

Figure 4 is a schematic representation of a front view of a device according to an example of the present disclosure. In this figure, the top engaging portion 114 comprises a top element protrusion 116 protruding from the top element cavity surface. The top element protrusion 116 of this example comprises a conical shape. The distal end of this example has a diameter between 10 mm and 20mm. In other examples, the top element protrusion may comprise a right circular cylindrical shape. The top element protrusion 116 increases the pressure applied by the top element 110 against the tennis ball 190. This may allow using elastic traction connectors configured to provide less traction force while exercising a similar pressure on the portion of the ball.

The top element 110 of this example further comprises an outer surface 117 opposite to the cavity 130. The outer surface 117 comprises a handle 118 configured to be handled to move the top element 110 towards the insertion position or towards the pressure position. The top element 110 may thus be easily moved upwards and downwards. Consequently, the users may operate the testing device in a simple manner.

In this figure, the base 120 comprises a base engaging portion 124 engaging the tennis ball 190. The base engaging portion 124 of this example comprises a recess having a concave surface. In fig. 4, the base engaging portion 124 defines a portion of a semi-sphere. The tennis ball 190 may be inserted in the semi-spherical recess of the base engaging portion 124 of this example.

As can be seen in fig. 4, the device 100 further comprises an adjusting element 25 configured to retain the ball within the base engaging portion of the base 120. The adjusting element 25 may be e.g. an additional deformable protrusion or an adjusting strip configured to surround a portion of the tennis ball.

The device 100 comprises a frame 160 movably connecting the base 120 with the top element 110. The frame 160 of this figure comprises a guide 161 to guide the movement of the top element 110 between the insertion position and the pressure position. The frame of this example is substantially rectangular or squared, i.e. comprises four lateral sides.

In this example, the frame comprises a plurality of columns extending between the base and the top element. The top element may thus be moved relative to these columns. These columns may be guides or rails to guide the up and down movement of the top element. In some examples, the guide is slidably connected to at least one of the base and the top element. In this example, the frame comprises four columns acting as a guide of the top element 110. Each of these columns is arranged at the corners between the lateral sides of the frame.

The columns according to the example of fig. 4 are substantially parallel to the movement of the top element 110. In this example, the top element 110 and the base 120 are substantially parallel to each other. The columns of the frame 160 are thus substantially perpendicular to both the base 120 and the top element 110. This configuration of the frame 160 allows the top element 110 to shift along a direction substantially parallel to the columns when the top element 110 is moved towards the base 120 by the effect of the traction of the elastic traction connector 140 or away from the base 120.

In this figure, the elastic traction connector 140 comprises four traction springs. Each of these traction springs is associated with a column. The columns may comprise a spring guide to avoid the bending or torsion of the traction springs.

The base may be rigidly connected to the frame and the top element may slide relative to the frame, e.g. relative to the guides arranged at the columns. Distance between the columns may define apertures to insert the tennis ball into the cavity. The frame may comprise one or more apertures for inserting the tennis ball. In this example, the frame 160 comprises a side cover 165. The side cover 165 may partially enclose the base 120. The side covers may be arranged at each side or at some of the sides of the frame. In some examples, one or more side covers may be hingedly connected to the columns to insert the tennis ball into the cavity.

In this figure, the frame 160 comprises a stopper 162 to limit a movement of the top element 110 relative to the base 120. In this example, the frame 160 comprises a plurality of stoppers 162 to limit the movement of the top element 110. The stoppers may be lower stoppers arranged below the top element 110 to limit the downwards movement of the top element 110. The stoppers may also be upper stoppers arranged above the top element 110 to limit the upward movement of the top element 110. The stoppers may be arranged at the columns of the frame. A stopper may be arranged at each of the columns of the frame.

In fig. 4, the measurement instrument 150 comprises magnifying glasses 152 to magnify the measurement of the deformation of a tennis ball 190 inserted in the device 100 providing better precision to the reading of the measurement. The measurement instrument 150 measures the displacement of the top element 110 relative to the base 120. Depending on the amount of displacement the tennis ball may be fit to play.

The device of fig. 4 also comprises an anchor 170 providing stability to the device which may be easily anchored to surfaces substantially flat. The anchor of this figure comprises a plurality of suction cups.

Figure 5 is a top view representation of a device according to an example of the present disclosure.

As can be seen in fig. 5, the frame 160 comprises a side cover 165 with a circular arc shape. The tennis ball may thus be inserted into the cavity through the aperture of the circular arc-shaped side cover. In other examples, the shape of the side cover may be e.g. rectangular or squared. The presence of the aperture in the shape of the side cover facilitates the insertion of the ball into the cavity.

In the example shown in fig. 5, the top element 110 is a cross shape. A player may benefit from this shape e.g. to hold the top element without the need of a specific top element handle or knob. According to the example shown in fig. 5, the elastic traction connector 140 comprises four traction springs connecting the top element to the frame.

In other examples, the top element is circular, rectangular, or squared.

A method for testing the status of a tennis ball comprising is also disclosed. The method comprises providing a device according to any of the examples herein disclosed.

The method comprises moving the top element to the insertion position by stretching the elastic traction connector. Stretching the elastic traction connector may comprise pulling the top element far away from the base. The top element is thus moved up to provide the cavity with sufficient space to accommodate a tennis ball.

Additionally, the method comprises inserting the tennis ball into the cavity of the device. For example, the tennis ball may be inserted into the cavity through the space between columns of the frame.

The method also comprises releasing the top element to move the top element from the insertion position to the pressure position by the effect of the traction exerted by the elastic traction connector. Therefore, the movement of the top element to the pressure position forces the ball against the base. The fact that the ball is forced against the base by the top element may cause a deformation of the ball which is a sign of the status of the ball.

Finally, the method comprises measuring a distance between the top element and the base when the ball is into the cavity of the device at the pressure position. The distance between the top element and the base corresponds to the amount of deformation of the ball under the stress exerted by the top element. The method may provide measuring qualitatively the status of the ball (indirect measurement of the distance) and/or measuring quantitatively the distance between the base and the top element. A qualitative measurement may be appreciated when the method provides a device comprising a qualitative indicator of the distance (e.g. a label). Furthermore, a method providing a device comprising a quantitative measurement instrument (e.g. a ruler) may give an exact measurement of the distance.

Although only a number of examples have been disclosed herein, other alternatives, modifications, uses and/or equivalents thereof are possible. Furthermore, all possible combinations of the described examples are also covered. The scope of the present invention is defined by the appended claims.

## Claims

1. Device (100) for testing a status of a tennis ball (190), comprising:
- a base (120);
- a top element (110) movably connected to the base (120);
- a cavity (130) between the base (120) and the top element (110) configured to receive the tennis ball (190); and
- a measurement instrument (150) for measuring a distance between the base (120) and the top element (110);
**characterised by**
- an elastic traction connector (140) movably connecting the base (120) to the top element (110), such that the top element (110) is movable between an insertion position for inserting the tennis ball (190) into the cavity (130) and a pressure position in which the top element (110) forces the tennis ball (190) against the base (120) by the effect of a traction exerted by the elastic traction connector (140).

2. The device (100) according to claim 1, wherein the elastic traction connector (140) comprises a plurality of traction springs.

3. The device (100) according to any of claims 1 or 2, wherein the elastic traction connector (140) comprises an elastic band.

4. The device (100) according to any of claims 1 - 3, wherein the base (120) comprises a base cavity surface (122) facing the cavity (130) and the top element (110) comprises a top element cavity surface (112) facing the cavity (130).

5. The device (100) according to claims 4, wherein the top element cavity surface (112) comprises a top engaging portion (114) configured to engage a portion of the tennis ball (190) facing the top element cavity surface (112).

6. The device (100) according to claim 5, wherein the top engaging portion (114) comprises a top element protrusion (116) configured to contact the portion of the tennis ball (190) facing the top element cavity surface (112) and to force the tennis ball (190) against the base cavity surface (122).

7. The device (100) according to any of claims 5 - 6, wherein the base engaging portion (124) comprises an adjusting element (25) configured to retain the tennis ball (190) within the base engaging portion (124).

8. The device (100) according to any of the preceding claims, further comprising an anchor (170) configured to anchor the base (120) to a substantially flat surface.

9. The device (100) according to claim 8, wherein the anchor (170) comprises a suction pad, a clamp and/or a screw.

10. The device (100) according to any of the preceding claims, wherein the measurement instrument (150) comprises a ruler and/or an indicator to indicate if the distance is equal to or greater than a measuring threshold.

11. The device (100) according to any of the preceding claims, further comprising a frame (160) movably connecting the base (120) with the top element (110), wherein the frame comprises a guide (160) to guide the top element (110) between the insertion position and the pressure position.

12. The device (100) according to claim 11, wherein the guide is slidably connected to at least one of the base (120) or the top element (110).

13. The device (100) according to any of claims 11 - 12, wherein the frame (160) comprises a stopper (162) to limit a movement of the top element (110) relative to the base (120).

14. The device (100) according to any of claims 11 - 13, wherein the frame (160) comprises one or more columns substantially parallel to the movement of the top element (110).

15. A method for testing a status of a tennis ball (190) comprising:
- providing a device (100) according to any of claims 1 - 14;
- moving the top element (110) to the insertion position by stretching the elastic traction connector (140);
- inserting the tennis ball (190) into the cavity (130);
- releasing the top element (110) to move the top element (110) from the insertion position to the pressure position by the effect of the traction exerted by the elastic traction connector (140), such that moving the top element (110) to the pressure position forces the tennis ball (190) against the base (120); and
- measuring a distance between the top element (110) and the base (120).

## Patentansprüche

1. Vorrichtung (100) zum Testen eines Status eines Tennisballs (190), umfassend:
- eine Basis (120);
- ein Deckelement (110), das beweglich mit der Basis (120) verbunden ist;
- einen Hohlraum (130) zwischen der Basis (120) und dem Deckelement (110), der dazu konfiguriert ist, den Tennisball (190) aufzunehmen; und
- ein Messinstrument (150) zum Messen eines Abstands zwischen der Basis (120) und dem Deckelement (110);
**gekennzeichnet durch**
- einen elastischen Zugverbinder (140), der die Basis (120) beweglich mit dem Deckelement (110) verbindet, so dass das Deckelement (110) zwischen einer Einführposition zum Einführen des Tennisballs (190) in den Hohlraum (130) und einer Druckposition, in welcher das Deckelement (110) den Tennisball (190) durch die Wirkung einer durch den elastischen Zugverbinder (140) ausgeübten Zugkraft gegen die Basis (120) drückt, beweglich ist.

2. Die Vorrichtung (100) nach Anspruch 1, wobei der elastische Zugverbinder (140) eine Vielzahl von Zugfedern umfasst.

3. Die Vorrichtung (100) nach einem der Ansprüche 1 oder 2, wobei der elastische Zugverbinder (140) ein elastisches Band umfasst.

4. Die Vorrichtung (100) nach einem der Ansprüche 1 bis 3, wobei die Basis (120) eine Basishohlraumfläche (122) umfasst, die dem Hohlraum (130) zugewandt ist, und das Deckelement (110) eine Deckelementhohlraumfläche (112) umfasst, die dem Hohlraum (130) zugewandt ist.

5. Die Vorrichtung (100) nach Anspruch 4, wobei die Deckelementhohlraumfläche (112) einen Deckeingriffsabschnitt (114) umfasst, der dazu konfiguriert ist, mit einem Abschnitt des Tennisballs (190) in Eingriff zu treten, der der Deckelementhohlraumfläche (112) zugewandt ist.

6. Die Vorrichtung (100) nach Anspruch 5, wobei der Deckeingriffsabschnitt (114) einen Deckelementvorsprung (116) umfasst, der dazu konfiguriert ist, den Abschnitt des Tennisballs (190), der der Deckelementhohlraumfläche (112) zugewandt ist, zu berühren und den Tennisball (190) gegen die Basishohlraumfläche (122) zu drücken.

7. Die Vorrichtung (100) nach einem der Ansprüche 5 bis 6, wobei der Basiseingriffsabschnitt (124) ein Einstellelement (25) umfasst, das dazu konfiguriert ist, den Tennisball (190) innerhalb des Basiseingriffsabschnitts (124) zu halten.

8. Die Vorrichtung (100) nach einem der vorhergehenden Ansprüche, ferner umfassend einen Anker (170), der dazu konfiguriert ist, die Basis (120) an einer im Wesentlichen flachen Oberfläche zu verankern.

9. Die Vorrichtung (100) nach Anspruch 8, wobei der Anker (170) einen Saugnapf, eine Klemme und/oder eine Schraube umfasst.

10. Die Vorrichtung (100) nach einem der vorhergehenden Ansprüche, wobei das Messinstrument (150) ein Lineal und/oder einen Zeiger umfasst, um anzuzeigen, ob der Abstand gleich oder größer als ein Messschwellenwert ist.

11. Die Vorrichtung (100) nach einem der vorhergehenden Ansprüche, ferner umfassend einen Rahmen (160), der die Basis (120) beweglich mit dem Deckelement (110) verbindet, wobei der Rahmen eine Führung (160) umfasst, um das Deckelement (110) zwischen der Einführposition und der Druckposition zu führen.

12. Die Vorrichtung (100) nach Anspruch 11, wobei die Führung verschiebbar mit mindestens einem von der Basis (120) oder dem Deckelement (110) verbunden ist.

13. Die Vorrichtung (100) nach einem der Ansprüche 11 bis 12, wobei der Rahmen (160) einen Anschlag (162) umfasst, um eine Bewegung des Deckelements (110) relativ zu der Basis (120) zu begrenzen.

14. Die Vorrichtung (100) nach einem der Ansprüche 11 bis 13, wobei der Rahmen (160) eine oder mehrere Säulen umfasst, die im Wesentlichen parallel zu der Bewegung des Deckelements (110) sind.

15. Ein Verfahren zum Testen eines Status eines Tennisballs (190), umfassend:
- bereitstellen einer Vorrichtung (100) nach einem der Ansprüche 1 bis 14;
- bewegen des Deckelements (110) in die Einführposition durch Dehnen des elastischen Zugverbinders (140);
- einführen des Tennisballs (190) in den Hohlraum (130);
- lösen des Deckelements (110), um das Deckelement (110) durch die Wirkung der durch den elastischen Zugverbinder (140) ausgeübten Zugkraft von der Einführposition in die Druckposition zu bewegen, sodass das Bewegen des Deckelements (110) in die Druckposition den Tennisball (190) gegen die Basis (120) drückt; und
- messen eines Abstands zwischen dem Deckelement (110) und der Basis (120).

## Revendications

1. Dispositif (100) pour tester un état d'une balle de tennis (190), comprenant :
- une base (120) ;
- un élément supérieur (110) relié de manière mobile à la base (120) ;
- une cavité (130) entre la base (120) et l'élément supérieur (110) configuré pour recevoir la balle de tennis (190) ; et
- un instrument de mesure (150) pour mesurer une distance entre la base (120) et l'élément supérieur (110) ;
**caractérisé par**
- un connecteur de traction élastique (140) reliant de manière mobile la base (120) à l'élément supérieur (110), de sorte que l'élément supérieur (110) est mobile entre une position d'insertion pour insérer la balle de tennis (190) dans la cavité (130) et une position de pression dans laquelle l'élément supérieur (110) force la balle de tennis (190) contre la base (120) sous l'effet d'une traction exercée par le connecteur de traction élastique (140).

2. Le dispositif (100) selon la revendication 1, dans lequel le connecteur de traction élastique (140) comprend une pluralité de ressorts de traction.

3. Le dispositif (100) selon l'une quelconque des revendications 1 ou 2, dans lequel le connecteur de traction élastique (140) comprend une bande élastique.

4. Le dispositif (100) selon l'une quelconque des revendications 1 à 3, dans lequel la base (120) comprend une surface de cavité de base (122) faisant face à la cavité (130) et l'élément supérieur (110) comprend une surface de cavité d'élément supérieur (112) faisant face à la cavité (130).

5. Le dispositif (100) selon la revendication 4, dans lequel la surface de cavité d'élément supérieur (112) comprend une partie d'engagement supérieure (114) configurée pour engager une partie de la balle de tennis (190) faisant face à la surface de cavité d'élément supérieur (112).

6. Le dispositif (100) selon la revendication 5, dans lequel la partie d'engagement supérieure (114) comprend une saillie d'élément supérieur (116) configurée pour entrer en contact avec la partie de la balle de tennis (190) faisant face à la surface de cavité d'élément supérieur (112) et pour forcer la balle de tennis (190) contre la surface de cavité de base (122).

7. Le dispositif (100) selon l'une quelconque des revendications 5 à 6, dans lequel la partie d'engagement de base (124) comprend un élément de réglage (25) configuré pour retenir la balle de tennis (190) à l'intérieur de la partie d'engagement de base (124).

8. Le dispositif (100) selon l'une quelconque des revendications précédentes, comprenant en outre un ancrage (170) configuré pour ancrer la base (120) à une surface essentiellement plate.

9. Le dispositif (100) selon la revendication 8, dans lequel l'ancrage (170) comprend une ventouse, une pince et/ou une vis.

10. Le dispositif (100) selon l'une quelconque des revendications précédentes, dans lequel l'instrument de mesure (150) comprend une règle et/ou un indicateur pour indiquer si la distance est égale ou supérieure à un seuil de mesure.

11. Le dispositif (100) selon l'une quelconque des revendications précédentes, comprenant en outre un cadre (160) reliant de manière mobile la base (120) à l'élément supérieur (110), dans lequel le cadre comprend un guide (160) pour guider l'élément supérieur (110) entre la position d'insertion et la position de pression.

12. Le dispositif (100) selon la revendication 11, dans lequel le guide est connecté de manière coulissante à au moins l'un de la base (120) ou de l'élément supérieur (110).

13. Le dispositif (100) selon l'une quelconque des revendications 11 à 12, dans lequel le cadre (160) comprend une butée (162) pour limiter un mouvement de l'élément supérieur (110) par rapport à la base (120).

14. Le dispositif (100) selon l'une quelconque des revendications 11 à 13, dans lequel le cadre (160) comprend une ou plusieurs colonnes essentiellement parallèles au mouvement de l'élément supérieur (110).

15. Un procédé pour tester un état d'une balle de tennis (190) comprenant :
- fournir un dispositif (100) selon l'une quelconque des revendications 1 à 14 ;
- déplacer l'élément supérieur (110) jusqu'à la position d'insertion en étirant le connecteur de traction élastique (140) ;
- insérer la balle de tennis (190) dans la cavité (130) ;
- libérer l'élément supérieur (110) pour déplacer l'élément supérieur (110) de la position d'insertion à la position de pression sous l'effet de la traction exercée par le connecteur de traction élastique (140), de sorte que le déplacement de l'élément supérieur (110) jusqu'à la position de pression force la balle de tennis (190) contre la base (120) ; et
- mesurer une distance entre l'élément supérieur (110) et la base (120).
